# EUROPÄISCHE PATENTSCHRIFT

(11) **EP 2 845 610 B1**
(45) Veröffentlichungstag und Bekanntmachung des Hinweises auf die Patenterteilung: **01.03.2017**
(21) Anmeldenummer: 14184324.3
(22) Anmeldetag: 10.09.2014
(51) Int. Cl.: A61L 2/08, B65B 55/08, B67C 7/00

(54) **Vorrichtung zur Strahlenabschirmung beim Sterilisieren von Behältnissen**
Apparatus for radiation shielding in sterilising containers
Dispositif pour protection contre les rayonnements de stérilisation de récipients

(30) Priorität: 10.09.2013 DE 202013104114 U
(43) Veröffentlichungstag der Anmeldung: 11.03.2015
(73) Patentinhaber: Krones AG, 93073 Neutraubling (DE)
(72) Erfinder: Scheuren, Hans, 93073 Neutraubling (DE); Knott, Josef, 93073 Neutraubling (DE); Neubauer, Michael, 93073 Neutraubling (DE)
(74) Vertreter: Bittner, Bernhard

(56) Entgegenhaltungen:
- EP-A1- 1 982 920
- EP-A2- 2 594 493
- DE-A1-102010 012 569
- US-A1- 2013 015 365

## Beschreibung

Die vorliegende Erfindung bezieht sich auf eine Vorrichtung zum Sterilisieren von Behältnissen. Im Bereich der Getränke herstellenden Industrie ist es seit langem bekannt, dass die abzufüllenden Behältnisse, insbesondere vor ihrer Befüllung, sterilisiert werden. Dazu wurden in der Vergangenheit üblicherweise chemische Substanzen wie Wasserstoffperoxid oder Peressigsäure eingesetzt. Der Nachteil dieser Sterilisation liegt darin, dass die chemischen Substanzen nach der jeweiligen Behandlung mit Sterilwasser aus den Behältnissen gespült werden müssen. Daher geht man in jüngerer Zeit auch dazu über, die Sterilisation der Behältnisse durch Bestrahlung und insbesondere durch Bestrahlung mit Elektronen oder Ladungsträgern durchzuführen. Dabei entsteht als unerwünschter Nebeneffekt unerwünschte Strahlung und insbesondere Röntgenstrahlung. Daher ist der Schutz der Umgebung und insbesondere des Bedieners der Anlage vor unerwünschter Strahlung von besonderer Bedeutung. Hierfür bedarf es einer Abschirmung, welche den Austritt der auftretenden Strahlungsarten nicht erlaubt.

Die aktuelle Praxis schirmt dabei insbesondere mit hoch dichten Materialien, wie etwa Blei, ab. Dieses Material ist jedoch aufgrund seiner Giftigkeit im Lebensmittelbereich und daher auch in einer zugehörigen Packmaschine unerwünscht. Weiterhin ist Blei mechanisch anfälliger als andere Materialien. Etwaige Verletzungen des Materials oder einfaches, zeitabhängiges Fließen führen zu möglichen Undichtigkeiten und somit zum Strahlenaustritt. In diesem Zusammenhang ist auch die Entsorgung des Bleis mit Zurückbau der Maschine zu berücksichtigen.

Aus der US 2013/0015365 A1 ist eine Vorrichtung zum Sterilisieren von Flaschen bekannt. Dabei soll der Herstellungsaufwand für die Abschirmung von Strahlung vermindert werden. Die DE 10 2010 012 569A1 beschreibt eine Vorrichtung zum Sterilisieren von Behältnissen wobei sowohl eine Sterilisierung einer Innenwandung von Behältnissen als auch eine Sterilisierung einer Außenwandung von Behältnissen ermöglicht ist.

Die EP 2 594 493 A2 offenbart eine Vorrichtung zur Innen- und Außensterilisation von Kunststoffbehältnissen mittels Ladungsträgerstrahlen. Aus der EP 1 982 920 A1 ist eine Vorrichtung zum Sterilsieren von Behältnissen bekannt, welche einen in Behältnisse einführbaren Behandlungskopf aufweist.

Daher werden als vorteilhaftere Abschirmung auch ausreichend dicke Stahl- und Gussabschirmungen mit höherer mechanischer Stabilität und Lebensmittelkonformität verwendet. Auch hier ist ein unveränderter Nachteil jedoch darin zu sehen, dass zur Beheizung der dicken Platten große Energiemengen benötigt werden. Eine solche Aufheizung ist beispielsweise vor Beginn der Sterilisation nötig, da zuvor die Oberflächen der Sterilisationsanlage entkeimt werden müssen. Dies kann beispielsweise mittels Wasserstoffperoxidgases bei 70° C geschehen. Bei Abschirmplatten, die ausreichend dick sind, um den Schutz des Bedieners der Anlage zu gewährleisten, benötigt eine Aufheizung hohe Energiemengen und lange Prozesszeiten.

Der vorliegenden Erfindung liegt daher die Aufgabe zu Grunde, bei einer weitreichenden Strahlenabschirmung und einer hohen Sicherheit den Energieverbrauch und die Prozesszeit im Falle einer Aufheizung zu reduzieren. Dabei sollen bleihaltige Oberflächen in der Nähe von Verpackungsmaterial vermieden und eine Korrosion der Abschirmung minimiert werden. Diese Aufgaben werden erfindungsgemäß durch die Gegenstände der unabhängigen Ansprüche erreicht. Vorteilhafte Ausführungsformen und Weiterbildungen sind Gegenstand der Unteransprüche.

Eine erfindungsgemäße Vorrichtung zum Sterilisieren von Behältnissen weist eine Transporteinrichtung auf, welche die Behältnisse entlang eines vorgegebenen Transportpfades transportiert. Weiterhin weist die Vorrichtung wenigstens eine Sterilisationseinrichtung auf, welche die Behältnisse während ihres Transports zu ihrer Sterilisation mit Ladungsträgern beaufschlagt.

Daneben weist die Vorrichtung eine Abschirmvorrichtung auf, welche dem Abschirmen von unerwünschter Strahlung und insbesondere von während des Sterilisationsprozesses und/oder während der Beaufschlagung der Kunststoffbehältnisse mit Elektronenstrahlung entstehender Strahlung dient. Vorteilhaft handelt es sich bei den Kunststoffbehältnissen um Kunststoffvorformlinge.

Diese Abschirmvorrichtung weist dabei wenigstens zwei Abschirmkörper auf. Diese Abschirmkörper sind so zueinander angeordnet, dass sich ein Abschirmkörper auf der dem Transportpfad zugewandten Seite befindet und ein Abschirmkörper auf der ihm abgewandten. Bei den Abschirmkörpern handelt es sich dabei vorteilhaft um Wandungen. Diese sind bevorzugt parallel zueinander angeordnet.

Die Abschirmkörper erstrecken sich dabei bevorzugt wenigstens abschnittsweise entlang des Transportpfades und/oder seitlich neben dem Transportpfad der Behältnisse. Unter einer Erstreckung entlang des Transportpfades muss dabei jedoch nicht zwangsläufig verstanden werden, dass die Abschirmkörper in gleicher Weise gekrümmt sind wie der Transportpfad. So wäre es beispielsweise auch möglich, dass ein Abschnitt der Abschirmvorrichtung, der sich entlang eines geradlinigen Abschnitts erstreckt, zur Abschirmung einer gekrümmten Transportbahn der Behältnisse dient. Es wäre dabei auch möglich, an bestimmten Stellen entlang des Transportpfades auf den dem Transportpfad zugewandten Abschirmkörper zu verzichten, wenn an diesen Stellen die Strahlenbelastung so gering ist, dass der dem Transportpfad abgewandte Abschirmkörper materialarm ausgeführt werden kann.

Erfindungsgemäß sind die Abschirmkörper zueinander thermisch isoliert. Vorteilhaft wird diese thermische Isolation mithilfe eines Spalts erreicht, der bevorzugt zumindest abschnittsweise zwischen den Abschirmkörpern verläuft. Dieser Spalt ist bevorzugt mit einem gasförmigen Medium wie beispielsweise Luft gefüllt. Denkbar sind dabei jedoch auch andere isolierende Materialien. Durch diese Isolation wird eine sandwichartige Bauweise erreicht, durch die bei einer Aufheizung der dem Transportpfad zugewandten Oberfläche, wie dies beispielsweise bei einer Sterilisation der Sterilisationsanlage nötig sein kann, nicht der massereiche, dem Transportpfad abgewandte Abschirmkörper miterhitzt werden muss, sondern nur der dem Transportpfad zugewandte Abschirmkörper. Auf diese Weise können Energieaufwand und Prozesszeiten gesenkt werden. Damit dient der dem Transportpfad bzw. den Behältnissen zugewandte Abschirmkörper auch zur Abtrennung des dem Transportpfad abgewandten Abschirmkörpers.

Vorteilhaft sind die Abschirmkörper miteinander verfügt und bevorzugt sind sie mit Schweißnähten, Schweißpunkten und/oder Schraubverbindungen oder besonders bevorzugt mit Steckverbindungen verfügt. Vorteilhaft wird hierdurch dem dem Transportpfad zugewandten Abschirmkörper im Vergleich zum abgewandten eine vorgegebene Beweglichkeit eingeräumt. Diese wird bevorzugt senkrecht zum Transportpfad eingeräumt und erlaubt insbesondere eine Ausdehnung des Abschirmkörpers, die insbesondere durch thermische Beanspruchung hervorgerufen wird. Bevorzugt sind die Abschirmkörper stationär bezüglich einander angeordnet, es wäre jedoch auch denkbar, dass sich wenigstens ein und bevorzugt der den Behältnissen zugewandte Abschirmkörper mit den Behältnissen mitbewegt.

Bei dem Abschirmkörper, der dem Transportpfad abgewandt ist, handelt es sich bevorzugt um ein Stahlblech oder eine Gussteilfläche. Die Dicke des Abschirmkörpers wird dabei so gewählt, dass eine sichere Strahlungsabschirmung erfolgt. Dazu weist er bevorzugt eine Dicke auf, welche zwischen 10 mm und 150 mm liegt, bevorzugt zwischen 20 mm und 120 mm und besonders bevorzugt zwischen 28 mm und 102 mm liegt. Bei der abzuschirmenden Strahlung kann es sich dabei beispielsweise um die ionisierende Strahlung zur Sterilisation, also insbesondere Elektronenstrahlung, oder um aus der Sterilisation resultierende Störstrahlung, wie beispielsweise Röntgenstrahlung, handeln.

Bei dem Abschirmkörper, der dem Transportpfad zugewandt ist, handelt es sich bevorzugt um ein gegenüber Sterilisationsmedien beständiges Blech. Dieses weist vorteilhaft eine Dicke von weniger als 10 mm, bevorzugt von weniger als 5 mm und besonders bevorzugt von weniger als 3 mm auf.

Vorteilhaft weist die Vorrichtung weiterhin eine Heizeinrichtung zum Erwärmen wenigstens eines Abschirmkörpers auf. Bevorzugt handelt es sich bei dem zu erwärmenden Abschirmkörper um den dem Transportpfad zugewandten Abschirmkörper. Die Aufheizung des Abschirmkörpers wird dabei vorteilhaft durch die Einleitung von einem erwärmten gasförmigen Medium durchgeführt. Es wäre jedoch auch die Verwendung einer elektronischen Heizeinrichtung denkbar. Bevorzugt kann jede Abschirmung daher ein Heizelement enthalten.

Durch die erfindungsgemäße thermische Isolation wird somit bevorzugt nur der dem Transportpfad zugewandte Abschirmkörper erwärmt, während der massereiche, dem Transportpfad abgewandte Abschirmkörper nicht erhitzt werden muss.

Bevorzugt ist daher zum Aufheizen der dem Transportpfad zugewandten Oberfläche der Abschirmvorrichtung nur die Wärmeenergie nötig, die für das Aufheizen des dem Transportpfades zugewandten Abschirmkörper nötig ist.

Bei einer weiteren vorteilhaften Ausführungsform weist die Vorrichtung eine Vielzahl von Sterilisationseinrichtungen auf. Bevorzugt ist dabei wenigstens eine Sterilisationseinrichtung zum Sterilisieren zumindest eines Bereichs der Innenwandung der Behältnisse vorgesehen. Vorteilhaft weist die die Vorrichtung auch eine Außenbeaufschlagungseinrichtung zum Sterilisieren zumindest eines Abschnitts einer Außenwandung der Behältnisse auf. Bevorzugt ist die Vorrichtung zur Außensterilisation der Behältnisse gegenüber der Bewegung der Behältnisse stationär angeordnet.

Bei einer weiteren vorteilhaften Ausführungsform weist die Vorrichtung eine Innenbehandlungseinrichtung auf und diese Innenbehandlungseinrichtung weist bevorzugt ein Strahlungselement auf, dessen Längsrichtung sich bevorzugt im Wesentlichen senkrecht zu dem Transportpfad der Behältnisse erstreckt, und welches bevorzugt durch eine Relativbewegung des Behältnisses gegenüber dem Strahlungselement durch eine Mündung der zu sterilisierenden Behältnisse hindurch in das Innere der zu sterilisierenden Behältnisse einführbar ist. Weiterhin weist bevorzugt diese Innenbehandlungseinrichtung eine Beschleunigungseinrichtung zum Beschleunigen von Ladungsträgern auf, sowie auch ein Austrittsfenster, welches derart beschaffen ist, dass es durch eine Mündung der Behältnisse hindurch in diese einführbar ist. Bevorzugt handelt es sich bei den Ladungsträgern um geladene Teilchen und insbesondere um Elektronen.

Vorteilhaft handelt es sich bei dem benannten Austrittsfenster um eine Folie und insbesondere um eine Titanfolie. Diese Folie weist dabei bevorzugt eine Dicke auf, welche zwischen 6 µm und 20 µm liegt, bevorzugt zwischen 8 µm und 16 µm und besonders bevorzugt zwischen 8 µm und 12 µm.

Bei einer vorteilhaften Ausführungsform sind innerhalb der Abschirmungsvorrichtung außerdem auch Sterilisationseinrichtungen vorgesehen, mittels denen eine Sterilisation von Teilen der Abschirmvorrichtung und von Anlagenteilen wie etwa von Greifklammern, Transportsternen und dergleichen vorgenommen werden kann, so dass eine Sterilisation insbesondere, aber nicht ausschließlich, bei einem Wiederanfahren der Anlage durchgeführt werden kann. Des Weiteren weist die Transporteinrichtung bevorzugt einen bewegbaren Träger auf, an dem eine Vielzahl von Halteelementen zum Halten der Behältnisse angeordnet ist. Bei den Halteelementen kann es sich insbesondere, aber nicht ausschließlich, um Greifelemente handeln, welche die Behältnisse im vorgegebenen Abschnitt, beispielsweise unterhalb deren Mündung bzw. unterhalb deren Tragring greifen können.

Die vorliegende Erfindung ist weiterhin auf eine Vorrichtung zur Strahlungsabschirmung in einer Sterilisationsvorrichtung von Behältnissen, die eine Transporteinrichtung, welche die Behältnisse entlang eines vorbestimmten Transportpfades transportiert, und eine Vielzahl von Sterilisationseinrichtungen aufweist, gerichtet, wobei die Vorrichtung mindestens zwei Abschirmkörper aufweist, die so zueinander angeordnet sind, dass sich ein Abschirmkörper auf der dem Transportpfad zugewandten Seite der Vorrichtung befindet und ein Abschirmkörper auf der ihm abgewandten und der dem Transportpfad zugewandte Abschirmkörper aufgeheizt werden kann. Es ist dabei denkbar, dass sich (wie auch bei der oben genannten Vorrichtung) die Abschirmwirkung der beiden Abschirmkörper deutlich unterscheiden und insbesondere der dem Transportpfad zugewandte Abschirmkörper lediglich eine geringe oder (nahezu) keine Abschirmwirkung bei Röntgenstrahlung hat.

Erfindungsgemäß sind die Abschirmkörper zueinander thermisch isoliert. Vorteilhaft wird diese thermische Isolation mithilfe eines Spalts erreicht, der bevorzugt zumindest abschnittsweise zwischen den Abschirmkörpern verläuft. Dieser Spalt ist bevorzugt mit einem gasförmigen Medium wie beispielsweise Luft gefüllt.

Bei einer vorteilhaften Ausgestaltung, sind die Abschirmkörper miteinander verfügt. Vorteilhaft sind die Abschirmkörper dabei mit Schweißnähten, Schweißpunkten, Schraubverbindungen oder besonders bevorzugt mit Steckverbindungen verfügt. Vorteilhaft wird hierdurch dem dem Transportpfad zugewandten Abschirmkörper im Vergleich zum abgewandten eine vorgegebene Beweglichkeit eingeräumt. Diese wird bevorzugt senkrecht zum Transportpfad und/oder senkrecht zu einer Oberfläche des Abschirmkörpers eingeräumt und erlaubt insbesondere eine Ausdehnung des Abschirmkörpers, die insbesondere durch thermische Beanspruchung hervorgerufen wird.

Bei den Abschirmkörpern handelt es sich dabei vorteilhaft um Wandungen. Diese sind bevorzugt parallel zueinander angeordnet.

Bevorzugt erstreckt sich der Transportpfad zwischen zwei dem Transportpfad zugewandten Abschirmkörpern hindurch (und bevorzugt auch zwischen zwei dem Transportpfad abgewandten Abschirmkörpern hindurch) dabei wird bevorzugt durch die besagten Abschirmkörper ein Kanal gebildet, innerhalb dessen der Transportpfad verläuft.

Weitere Vorteile und Ausführungsformen ergeben sich aus den beigefügten Zeichnungen. Darin zeigen:
- Fig. 1: eine Darstellung einer Vorrichtung zum Sterilisieren von Behältnissen und
- Fig. 2: eine Detaildarstellung der Abschirmvorrichtung.

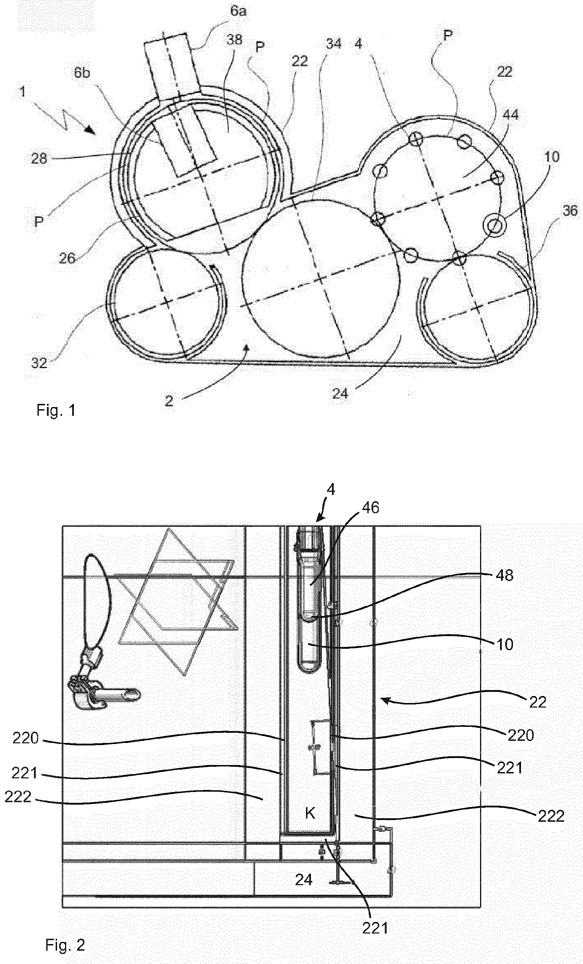

Fig. 1 zeigt eine Darstellung einer Vorrichtung 1 zum Sterilisieren von Behältnissen 10. Dabei werden die Behältnisse 10 der Vorrichtung 1 über eine Zuführöffnung (nicht gezeigt) und eine Zuführeinrichtung 32, die hier als Zuführstern ausgeführt ist, zugeführt. Anschließend erfolgt eine Außensterilisation der Behältnisse 10. Diese werden dabei auf einem kreisförmigen Transportpfadabschnitt vorbei an zwei Außen-Sterilisationseinrichtungen 6a und 6b geführt, welche eine Außenoberfläche der Behältnisse mit Ladungsträgern und insbesondere Elektronen bestrahlen. Das Bezugszeichen 38 kennzeichnet grob schematisch ein Transportelement wie einen Transportstern, der zum Transportieren der Behältnisse während deren Außensterilisation dient. Dieses Transportelement kann dabei einen drehbaren Träger aufweisen, an dem eine Vielzahl von Halteeinrichtungen zum Halten der Behältnisse angeordnet ist. Der Transportpfad der Behältnisse wird in diesem Bereich sowohl von der Wandung 22 nach außen hin als auch von der Wandung 26 nach innen hin begrenzt.

Anschließend werden die Behältnisse mit einem Transportelement 34 zu einer Innenbehandlungseinrichtung transportiert. Bei diesem Transportelement 34 kann es sich um einen Transportstern handeln, der auch einen Teilungsverzug zwischen den einzelnen Behältnissen 10 ermöglicht.

Das Bezugszeichen 4 bezieht sich auf zweite Sterilisationseinrichtungen, die an einem gemeinsamen und drehbaren Träger 44 angeordnet sind. Diese zweiten Sterilisationseinrichtungen 4 weisen dabei jeweils stangenartige Körper auf, die in einen Innenraum der Behältnisse einführbar sind, um diese so mit den Ladungsträgern zu beaufschlagen. Diese stangenartigen Körper weisen dabei jeweils an ihrem unteren Ende ein Austrittsfenster auf, durch welches hindurch die Ladungsträger aus diesen stangenartigen Körpern austreten können. Das Bezugszeichen 36 kennzeichnet eine Abführeinrichtung, welche die sterilisierten Behältnisse 10 wieder aus der Vorrichtung 1 abführt. Auch diese Abführeinrichtung 36 kann dabei als Transportstern ausgebildet sein. Das Bezugszeichen 2 bezieht sich auf die Transporteinrichtung in ihrer Gesamtheit, welche hier die oben beschriebenen einzelnen Transportelemente aufweist.

Um die Röntgenstrahlung, die sowohl bei der Außensterilisation als auch bei der Innensterilisation entsteht, abzuschirmen, weist die Vorrichtung eine Abschirmvorrichtung 22 auf, welche hier den vollständigen Transportpfad P der Behältnisse 10 umgibt. Nach oben hin wird die Strahlung durch einen Deckel 28 abgeschirmt und nach unten hin durch eine Bodenwandung 24.

Fig. 2 zeigt eine detailliertere Darstellung der Abschirmvorrichtung 22. Die Abschirmvorrichtung 22 weist dabei jeweils zwei Abschirmkörper 220 und 222 auf, wobei der Abschirmkörper 220 dem Transportpfad der Behältnisse 10 zugewandt und der Abschirmkörper 222 dem Transportpfad abgewandt ist. Der Transportpfad verläuft dabei innerhalb des Kanals K, der von zwei Abschirmkörpern 220 begrenzt wird. Der Abschirmkörper 220 weist dabei eine geringere Dicke als der Abschirmkörper 222 auf. Dadurch ist es möglich, die Zeit zum Erhitzen der Wandungen, die dem Transportpfad der Kunststoffvorformlinge zugewandt sind, zu verkürzen. Die Wandungen werden insbesondere auf eine Temperatur erhitzt, die oberhalb von 50°C, besonders bevorzugt oberhalb 60°C und insbesondere in einem Bereich von ca. 70°C liegt. Zwischen den Abschirmkörpern ist ein isolierender Spalt 221 zu erkennen. Es ist jedoch denkbar, dass der Spalt 221 durch ein Material zur thermischen Isolation ersetzt wird. Daneben sind auch die Bodenwandung 24 und die Innensterilisationseinrichtung 4 einschließlich des Strahlungselements 46 mit Austrittsfenster 48 abgebildet.

Der erfindungsgemäße Vorrichtung 1 kann eine Umformungseinrichtung (nicht gezeigt) nachgestellt sein, welche die Kunststoffvorformlinge zu den Kunststoffbehältnissen umformt. Diese Umformungseinrichtung kann dabei eine Transporteinrichtung aufweisen, wie etwa ein Blasrad, an dem eine Vielzahl von Umformungsstationen angeordnet ist. Weiterhin kann dieser Umformungseinrichtung noch ein Ofen vorangestellt sein, welcher die Kunststoffvorformlinge vor deren Expansion erwärmt. Die hier beschriebene Sterilisationsvorrichtung befindet sich vorteilhaft zwischen diesem Ofen und der Umformungseinrichtung. Damit werden hier bevorzugt die bereits erwärmten Kunststoffvorformlinge sterilisiert und anschließend zu den Kunststoffbehältnissen expandiert. Bevorzugt sind um die Sterilisationsvorrichtung Wandungen angeordnet, wobei die Wandungen aus Abschirmkörpern ausgebildet sind. Der der Sterilisationsvorrichtung zugewandte Abschirmkörper ist dabei dünner in seiner Dicke ausgestaltet, als der der Sterilisationsvorrichtung abgewandte Abschirmkörper. Die Abschirmkörper sind durch einen Luftspalt bevorzugt thermisch isolierend zueinander angeordnet.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind.

### Bezugszeichenliste

- P: Transportpfad
- K: Kanal
- 1: Vorrichtung
- 2: Transporteinrichtung
- 4: Sterilisationseinrichtungen/Innensterilisationseinrichtung
- 6a: Sterilisationseinrichtung
- 6b: Sterilisationseinrichtung
- 10: Behältnisse
- 22: Wandung/Abschirmvorrichtung
- 24: Bodenwandung
- 26: Wandung
- 28: Deckel
- 32: Zuführeinrichtung
- 34: Transportelement
- 38: Transportelement
- 44: Träger
- 46: Strahlungselement
- 48: Austrittsfenster
- 220: Abschirmkörper
- 221: isolierender Spalt
- 222: Abschirmkörper

## Patentansprüche

1. Vorrichtung (1) zum Sterilisieren von Behältnissen (10) mit einer Transporteinrichtung (2), welche die Behältnisse (10) entlang eines vorbestimmten Transportpfades (P) transportiert, mit einer Sterilisationseinrichtung (4, 6a, 6b), welche wenigstens einen Bereich der Behältnisse (10) im Rahmen der Sterilisation mit Ladungsträgern beaufschlagt, mit einer Abschirmvorrichtung (22) zur Abschirmung von Strahlen gegenüber der Umgehung, die mindestens zwei Abschirmkörper (220, 222) aufweist, die so zueinander angeordnet sind, dass sich ein Abschirmkörper (220) auf der dem Transportpfad (P) zugewandten Seite der Abschirmvorrichtung befindet und ein Abschirmkörper (222) auf der ihm abgewandten,
**dadurch gekennzeichnet, dass**
die Abschirmkörper zueinander thermisch isoliert sind.

2. Vorrichtung nach Anspruch 1,
**dadurch gekennzeichnet, dass**
es sich bei den Abschirmkörpern (220, 222) um Wandungen handelt, welche sich entlang des Transportpfades (P) erstrecken und/oder die Abschirmkörper (220, 222) parallel zueinander verlaufen.

3. Vorrichtung nach wenigstens einem der vorangegangenen Ansprüche,
**dadurch gekennzeichnet, dass**
die Abschirmkörper (220, 222) mithilfe eines Spalts (221), der zumindest abschnittsweise zwischen den Abschirmkörpern verläuft, thermisch isoliert sind,.

4. Vorrichtung nach wenigstens einem der vorangegangenen Ansprüche,
**dadurch gekennzeichnet, dass**
die Abschirmkörper (220, 222) miteinander verfügt sind und mit Schweißnähten, Schweißpunkten, Schraubverbindungen oder mit Steckverbindungen verfügt sind.

5. Vorrichtung nach wenigstens einem der vorangegangenen Ansprüche,
**dadurch gekennzeichnet, dass**
der dem Transportpfad (P) zugewandte Abschirmkörper (220) im Vergleich zum abgewandten (222) eine vorgegebene Beweglichkeit senkrecht zum Transportpfad aufweist.

6. Vorrichtung nach wenigstens einem der vorangegangenen Ansprüche,
**dadurch gekennzeichnet, dass**
es sich bei dem dem Transportpfad (P) abgewandten Abschirmkörper (222) um ein Stahlblech oder eine Gussteilfläche handelt und/oder es sich bei dem dem Transportpfad (P) zugewandten Abschirmkörper (220) um ein gegenüber Sterilisationsmedien beständiges Blech handelt.

7. Vorrichtung nach wenigstens einem der vorangegangenen Ansprüche,
**dadurch gekennzeichnet, dass**
der dem Transportpfad (P) abgewandte Abschirmkörper (222) 10-150mm, dick ist und/oder der dem Transportpfad (P) zugewandte Abschirmkörper (220) weniger als 10 mm, dick ist.

8. Vorrichtung nach wenigstens einem der vorangegangenen Ansprüche,
**dadurch gekennzeichnet, dass**
es sich bei der abzuschirmenden Strahlung um ionisierende Strahlung zur Sterilisation der Behältnisse (10) oder um aus der Sterilisation resultierende Störstrahlung handelt.

9. Vorrichtung nach wenigstens einem der vorangegangenen Ansprüche,
**dadurch gekennzeichnet, dass**
eine Heizeinrichtung zum Erwärmen wenigstens eines Abschirmkörpers (220, 222) vorgesehen ist.

10. Vorrichtung nach Anspruch 9,
**dadurch gekennzeichnet, dass**
es sich bei dem zu erwärmenden Abschirmkörper um den dem Transportpfad zugewandten Abschirmkörper (220) handelt.

11. Vorrichtung nach wenigstens einem der vorangegangenen Ansprüche,
**dadurch gekennzeichnet, dass**
die Anlage eine Vielzahl von Sterilisationseinrichtungen (4, 6a, 6b) aufweist, wobei die Sterilisationseinrichtung (4) wenigstens einen Bereich der Innenwandung der Behältnisse (10) sterilisiert.

12. Vorrichtung nach Anspruch 11,
**dadurch gekennzeichnet, dass**
die Sterilisationseinrichtung (4) ein Strahlungselement (46) aufweist, dessen Längsrichtung sich im Wesentlichen senkrecht zu dem Transportpfad (P) der Behältnisse (10) erstreckt, und welches durch eine Relativbewegung des Behältnisses (10) gegenüber dem Strahlungselement durch eine Mündung der zu sterilisierenden Behältnisse (10) hindurch in das Innere des zu sterilisierenden Behältnisses (10) einführbar ist.

13. Vorrichtung nach wenigstens einem der vorangegangenen Ansprüche,
**dadurch gekennzeichnet, dass**
die Transporteinrichtung (2) Greifelemente aufweist.

14. Vorrichtung zur Strahlungsabschirmung (22) in einer Sterilisationsvorrichtung (1) von Behältnissen (10), die eine Transporteinrichtung (2), welche die Behältnisse (10) entlang eines vorbestimmten Transportpfades (P) transportiert, und eine Vielzahl von Sterilisationseinrichtungen (4, 6a, 6b) aufweist, wobei die Vorrichtung mindestens zwei Abschirmkörper (220, 222) aufweist, die so zueinander angeordnet sind, dass sich ein Abschirmkörper (220) auf der dem Transportpfad (P) zugewandten Seite der Vorrichtung befindet, wobei eine Heizeinrichtung zum Erwärmen wenigstens eines Abschirmkörpers (220, 222) vorgesehen ist und ein Abschirmkörper (222) auf der ihm abgewandten und der dem Transportpfad zugewandte Abschirmkörper (220) aufgeheizt werden kann,
**dadurch gekennzeichnet, dass**
die Abschirmkörper zueinander thermisch isoliert sind.

## Claims

1. An apparatus (1) for the sterilization of containers (10) with a conveying device (2) which conveys the containers (10) along a pre-determined conveying path (P), with a sterilization device (4, 6a, 6b) which acts upon at least one area of the containers (10) with charge carriers in the course of the sterilization, with a screening apparatus (22) for screening off beams from the environment, which has at least two screening bodies (220, 222) which are arranged with respect to each other in such a way that one screening body (220) is situated on the side of the screening apparatus facing the conveying path (P) and one screening body (222) is situated on the one facing away from it,
**characterized in that**
the screening bodies are thermally insulated from each other.

2. An apparatus according to claim 1,
**characterized in that**
the screening bodies (220, 222) are walls which extend along the conveying path (P) and/ or the screening bodies (220, 222) extend parallel to each other.

3. An apparatus according to at least one of the preceding claims,
**characterized in that**
the screening bodies (220, 222) are thermally insulated from each other with the aid of a gap (221) which extends at least locally between the screening bodies.

4. An apparatus according to at least one of the preceding claims,
**characterized in that**
the screening bodies (220, 222) are joined to each other and they are joined by welding seams, spot welds, screw connections or by plug-in connections.

5. An apparatus according to at least one of the preceding claims,
**characterized in that**
the screening body (220) facing the conveying path (P) has a pre-set movability at a right angle to the conveying path as compared with the one (222) facing away.

6. An apparatus according to at least one of the preceding claims,
**characterized in that**
the screening body (222) which faces away from the conveying path (P) is a steel sheet or the face of a casting and/ or the screening body (220) facing the conveying path (P) is a metal sheet which is resistant to sterilization media.

7. An apparatus according to at least one of the preceding claims,
**characterized in that**
the screening body (222) facing away from the conveying path (P) is from 10 to 150 mm thick, and/ or the screening body (220) facing the conveying path (P) is less than 10 mm thick.

8. An apparatus according to at least one of the preceding claims,
**characterized in that**
the radiation to be screened off is ionizing radiation for the sterilization of the containers (10) or interference radiation resulting from the sterilization.

9. An apparatus according to at least one of the preceding claims,
**characterized in that**
a heating device is provided for heating at least one screening body (220, 222).

10. An apparatus according to claim 9,
**characterized in that**
the screening body to be heated is the screening body (220) facing the conveying path.

11. An apparatus according to at least one of the preceding claims,
**characterized in that**
the plant has a plurality of sterilization devices (4, 6a, 6b) wherein the sterilization device (4) sterilizes at least one region of the inner wall of the containers (10).

12. An apparatus according to claim 11,
**characterized in that**
the sterilization device (4) has a radiation element (46), the longitudinal direction of which extends substantially at a right angle to the conveying path (P) of the containers (10) and which is capable of being introduced into the interior of the container (10) to be sterilized by a relative movement of the container (10) with respect to the radiation element through an aperture in the containers (10) to be sterilized.

13. An apparatus according to at least one of the preceding claims,
**characterized in that**
the conveying device (2) has gripping elements.

14. An apparatus for the screening off (22) of radiation in an apparatus (1) for the sterilization of containers (10), which has a conveying device (2), which conveys the containers (10) along a pre-determined conveying path (P), and a plurality of sterilization devices (4, 6a, 6b), wherein the apparatus having at least two screening bodies (220, 222) which are arranged with respect to each other in such a way that one screening body (220) is situated on the side of the apparatus facing the conveying path (P) wherein a heating device for heating at least one of the screening bodies (220, 222) is provided and which can heat up one screening body (222) which is facing away from it and one screening body (220) which is facing the conveying path,
**characterized in that**
the screening bodies are thermally insulated from each other.

## Revendications

1. Dispositif (1) servant à stériliser des récipients (10) comprenant un système de transport (2), qui transporte les récipients (10) le long d'un chemin de transport (P) prédéterminé, comprenant un système de stérilisation (4, 6a, 6b), qui soumet au moins une zone des récipients (10), dans le cadre de la stérilisation, à l'action de porteurs de charges, comprenant un dispositif de protection (22) servant à protéger des rayons par rapport à l'extérieur, lequel présente au moins deux corps de protection (220, 222), qui sont disposés l'un par rapport à l'autre de telle manière qu'un corps de protection (220) se trouve sur le côté, tourné vers le chemin de transport (P), du dispositif de protection et qu'un corps de protection (222) se trouve sur le côté opposé,
**caractérisé en ce**
**que** les corps de protection sont isolés thermiquement les uns des autres.

2. Dispositif selon la revendication 1,
**caractérisé en ce**
**que** les corps de protection (220, 222) sont des parois, qui s'étendent le long du chemin de transport (P), et/ou en ce que les corps de protection (220, 222) s'étendent de manière parallèle les uns par rapport aux autres.

3. Dispositif selon au moins l'une quelconque des revendications précédentes,
**caractérisé en ce**
**que** les corps de protection (220, 222) sont isolés thermiquement au moyen d'une fente (221), qui s'étend au moins par endroits entre les corps de protection.

4. Dispositif selon au moins l'une quelconque des revendications précédentes,
**caractérisé en ce**
**que** les corps de protection (220, 222) sont assemblés les uns aux autres et sont assemblés par des cordons de soudure, des points de soudure, des systèmes de liaison par vissage ou par des systèmes de liaison par emboîtement.

5. Dispositif selon au moins l'une quelconque des revendications précédentes,
**caractérisé en ce**
**que** le corps de protection (220) tourné vers le chemin de transport (P) présente, en comparaison avec le corps de protection (222) opposé, une mobilité prédéfinie de manière perpendiculaire par rapport au chemin de transport.

6. Dispositif selon au moins l'une quelconque des revendications précédentes,
**caractérisé en ce**
**que** le corps de protection (222) opposé au chemin de transport (P) est une tôle en acier ou une surface de pièce de coulée, et/ou en ce que le corps de protection (220) tourné vers le chemin de transport (P) est une tôle résistante par rapport aux milieux de stérilisation.

7. Dispositif selon au moins l'une quelconque des revendications précédentes,
**caractérisé en ce**
**que** le corps de protection (222) opposé au chemin de transport (P) présente une épaisseur de 10 - 150 mm, et/ou en ce que le corps de protection (220) tourné vers le chemin de transport (P) présente une épaisseur inférieure à 10 mm.

8. Dispositif selon au moins l'une quelconque des revendications précédentes,
**caractérisé en ce**
**que** le rayonnement protecteur est un rayonnement ionisant servant à stériliser les récipients (10) ou un rayonnement parasite résultant de la stérilisation.

9. Dispositif selon au moins l'une quelconque des revendications précédentes,
**caractérisé en ce**
**qu'**un système de chauffage est prévu afin de réchauffer au moins un corps de protection (220, 222).

10. Dispositif selon la revendication 9,
**caractérisé en ce**
**que** le corps de protection à réchauffer est le corps de protection (220) tourné vers le chemin de transport.

11. Dispositif selon au moins l'une quelconque des revendications précédentes,
**caractérisé en ce**
**que** l'installation présente une pluralité de systèmes de stérilisation (4, 6a, 6b), le système de stérilisation (4) stérilisant au moins une zone de la paroi intérieure des récipients (10).

12. Dispositif selon la revendication 11,
**caractérisé en ce**
**que** le système de stérilisation (4) présente un élément de rayonnement (46), dont le sens longitudinal s'étend sensiblement de manière perpendiculaire par rapport au chemin de transport (P) des récipients (10) et qui peut être introduit par un déplacement relatif du récipient (10) par rapport à l'élément de rayonnement à travers une embouchure des récipients (10) à stériliser à l'intérieur du récipient (10) à stériliser.

13. Dispositif selon au moins l'une quelconque des revendications précédentes,
**caractérisé en ce**
**que** le système de transport (2) présente des éléments de préhension.

14. Dispositif servant à protéger contre les rayonnements (22) dans un dispositif de stérilisation (1) de récipients (10), qui présente un système de transport (2), qui transporte les récipients (10) le long d'un chemin de transport (P) prédéterminé, et une pluralité de systèmes de stérilisation (4, 6a, 6b), le dispositif présentant au moins deux corps de protection (220, 222), qui sont disposés les uns par rapport aux autres de telle sorte qu'un corps de protection (220) se trouve sur le côté, tourné vers le chemin de transport (P), du dispositif, un système de chauffage servant à réchauffer au moins un corps de protection (220, 222) étant prévu et un corps de protection (222) se trouvant sur le côté opposé et le corps de protection (220) tourné vers le chemin de transport pouvant être chauffé,
**caractérisé en ce**
**que** les corps de protection sont isolés thermiquement les uns par rapport aux autres.
